Europäisches Patentamt

European Patent Office

Office européen des brevets

(19)

(11) Publication number: 0 142 268
A1

(12) EUROPEAN PATENT APPLICATION

(21) Application number: 84306934.5

(22) Date of filing: 11.10.84

(51) Int. Cl.⁴: C 12 N 15/00
C 12 P 21/02
//C07K13/00, C12R1:865

(30) Priority: 18.10.83 GB 8327880

(43) Date of publication of application:
22.05.85 Bulletin 85/21

(84) Designated Contracting States:
CH DE FR GB IT LI SE

(71) Applicant: AJINOMOTO CO., INC.
5-8, Kyobashi 1-chome, Chuo-ku
Tokyo 104(JP)

(71) Applicant: JAPANESE FOUNDATION FOR CANCER
RESEARCH
37-1, Kamiikebukuro 1-chome
Toshima-ku Tokyo(JP)

(72) Inventor: Taniguchi, Tadatsugu
No. 4-27-12-303, Tagara-chu
Nerima-ku Tokyo(JP)

(72) Inventor: Matsui, Hiroshi
No. 1-19-16-101, Namiki Kanazawa-ku
Yokohama-shi Kanagawa-ken(JP)

(72) Inventor: Hamuro, Junji
No. 241-32, Fukaya-cho Totsuka-ku
Kanagawa-ken(JP)

(72) Inventor: Sato, Takaaki
No. 2-20-8, Kannon Kawasaki-ku
Kawasaki-shi Kanagawa-ken(JP)

(72) Inventor: Sano, Konosuke
No. 1-35-9, Uohara
Shibuya Tokyo(JP)

(74) Representative: Bond, Bentley George et al,
Haseltine Lake & Co. 28 Southampton Buildings
Chancery Lane
London WC2A 1AT(GB)

(54) Saccharomyces cerevisiae possessing gene coding for interleukin-2 polypeptide and method for producing interleukin-2 using the yeast.

(57) There is disolved a cell of Saccharomyces cerevisiae transformed with a recontinant DNA comprising a gene coding for a polypeptide possessing the activity of interleukin-2 and a vector DNA capable of propagating in said cell, the coding sequence of said gene being located at a position downstream of a promoter sequence.

EP 0 142 268 A1

-1-

# SACCHAROMYCES CEREVISIAE POSSESSING GENE CODING FOR INTERLEUKIN-2 POLYPEPTIDE AND METHOD FOR PRODUCING INTERLEUKIN-2 USING THE YEAST

## BACKGROUND OF THE INVENTION

### Field of the Invention

The present invention relates to Saccharomyces cerevisia possessing gene coding for interleukin-2 polypeptide and method for producing interleukin-2 using the yeast.

### Brief Description of the Prior Art

Interleukin 2 (hereinafter referred to as "IL-2") formerly referred to as T cell growth factor, is a soluble protein (generally known as "lymphokine"), and is produced from T cells activated with a lectin or an antigen (Morgan. D.A., et al., Science, 193, 1007-1008 (1976), Gillis, S. et al., J. Immunol., 120, 2027-2033 (1978). Interleukin 2 (IL-2) is capable of modulating lymphocyte reactivity and promoting the in vitro long-term cultures of antigen specific effector T-lymphocytes (Gillis. S. et al., Nature 268, 154-156 (1977)). IL-2 is also known to manifest other relevant biological activities such as enhancement of thymocyte mitogenesis (Chen, B.M. et al., Cell. Immunol., 22, 211-224, (1977)), Shaw, J. et al., J. Immunol. 120, 1967-1973, (1978)), induction of cytotoxic T cell reactivity (Wagner, H. et al, Nature, 284, 278-280, (1980)) and anti-SRBC plaque forming cell responses (Gillis, S. et al., J. Exp. Med., 149, 1960-1968, (1979) in cultures of nude mouse spleen cells.   Accordingly, this lymphocyte regulatory substance is useful in potentiating humoral and cellular immune responses and in restoring immune deficient state to a normal humoral and cellular immune state.   These identified immunological activities of IL-2 strongly indicate that IL-2 is useful for medical immunotherapy against immunological disorders including neoplastic diseases, bacterial or viral infections, immune deficient diseases, autoimmune diseases etc. (Pagermaster, B. et al., Adv. Immunopharm., 507, (1980)).

In the pending European patent application of the same assignee as the present application (European Patent Application No. 83101035.0 = U.S. Patent Application serial No. 463,496), genes coding for IL-2 polypeptide, recombinant DNA carrying the genes, cells possessing the recombinant DNA, and methods for producing IL-2 using the cells has been disclosed.   The pending European patent application also suggests that the gene coding for IL-2 polypeptide can be incorporated into cells of Saccharomyces cerevisiae after the gene has been combined with a vector DNA capable of propagating in cells of Saccharomyces cerevisiae, and the genetic information of the incorporated gene can be expressed to produce IL-2.

SUMMARY OF THE INVENTION

The inventors have now succeeded in obtaining the cell of Saccharomyces cerevisiae transformed with a recombinant DNA comprising a gene coding for an IL-2 polypeptide and a vector DNA capable of propagating in the cell, the coding sequence of the gene being located at a position downstream of a promoter sequence.   The transformed Saccharomyces cerevisiae could produce IL-2 expressing the genetic information of the gene
incorporated in the cell.

BRIEF DESCRIPTION OF THE DRAWINGS

Figure 1 shows a restriction endonuclease cleavage
map of a cloned gene coded to produce a polypeptide
which possesses the activity of IL-2 (hereinafter
referred to as "IL-2 polypeptide").

Figure 2 (a) shows the base sequence of the cloned
gene.

Figure 2(b) shows Amino Acid Sequences I and II
of the polypeptide which possess IL-2 activity.

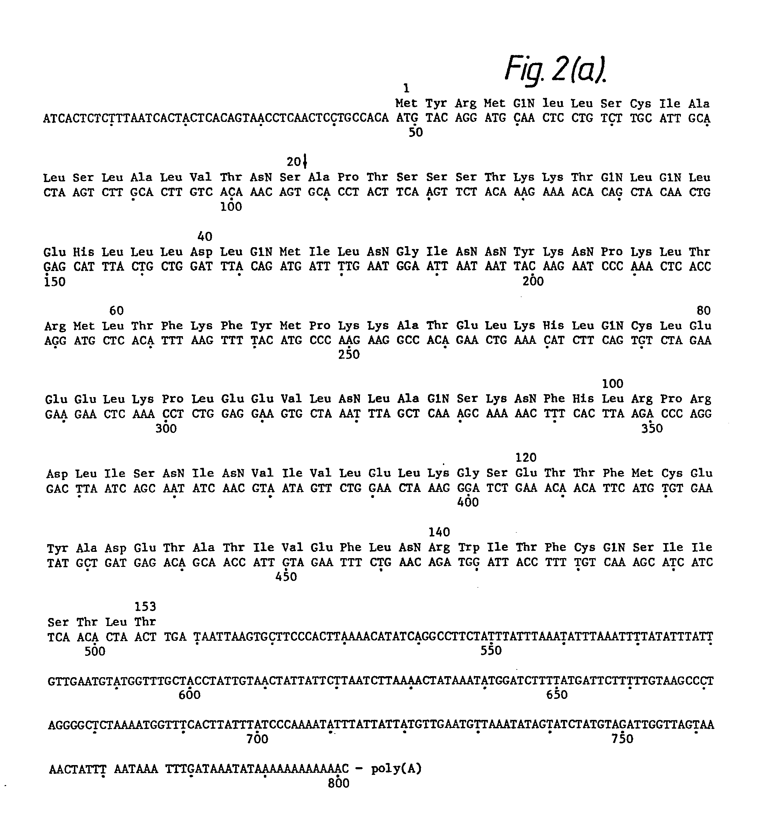

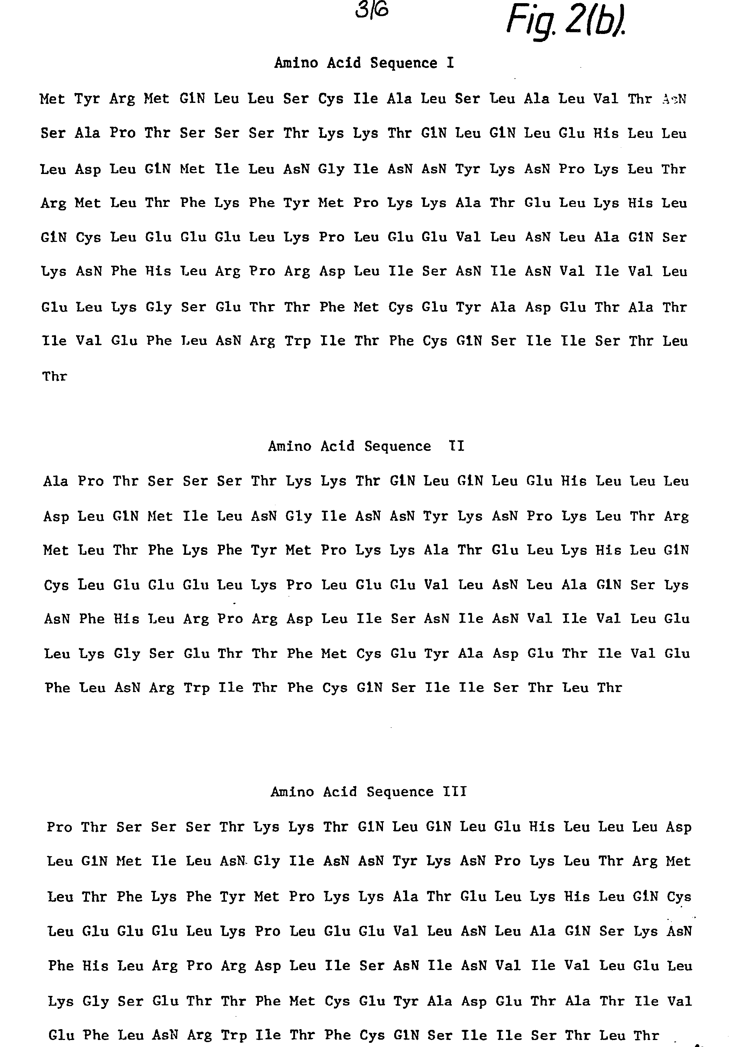

Figure 2 shows the plasmid vector pTrS-3.

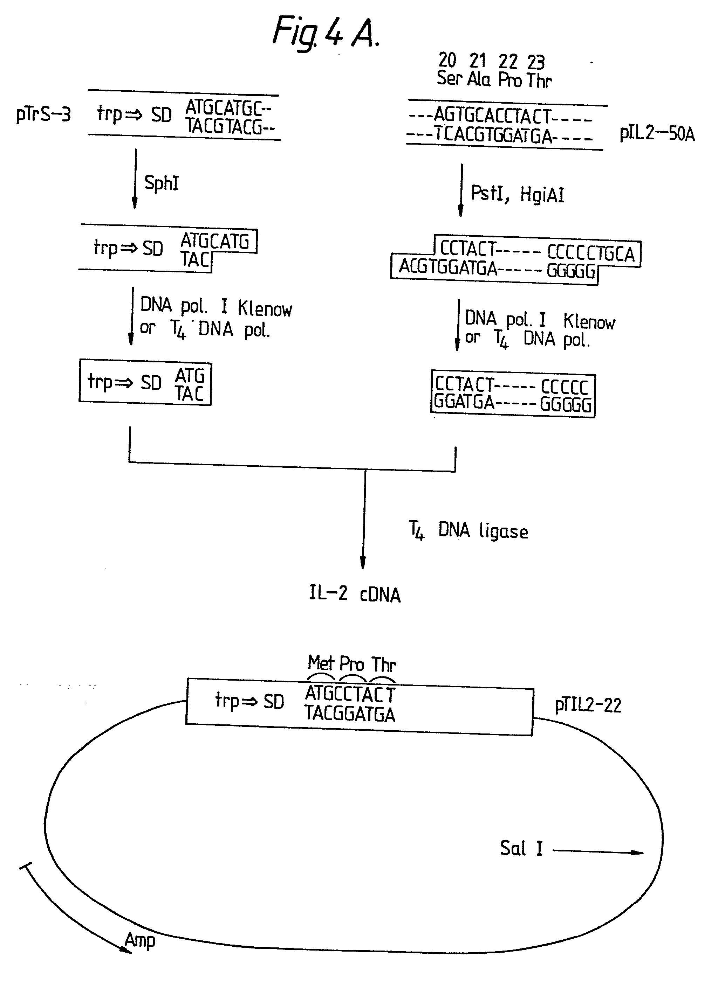

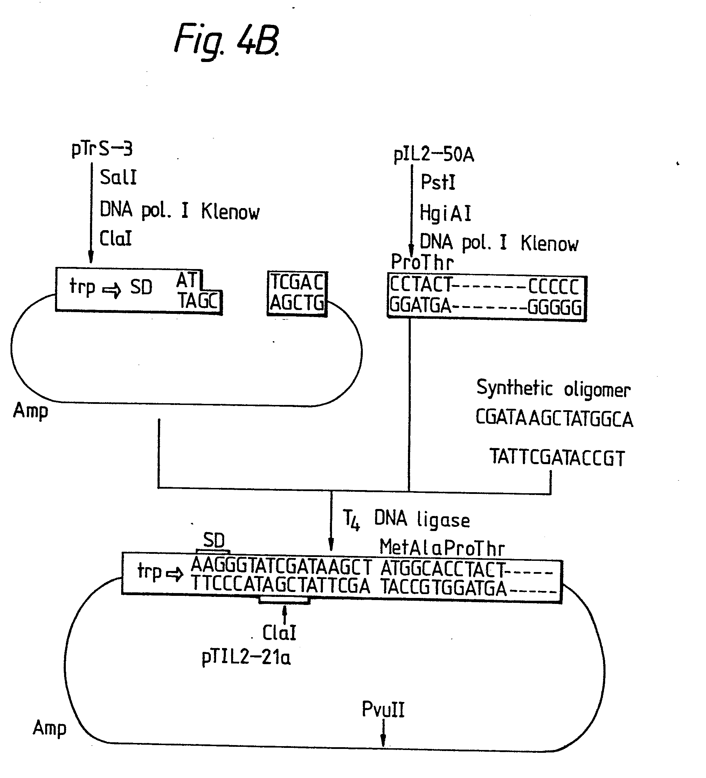

Figure 4(a) and 4(b) are flow charts showing the
construction of recombinant DNAs (pTIL 2-22, pTIL2-21,)
using pTrS-3 as a vector.

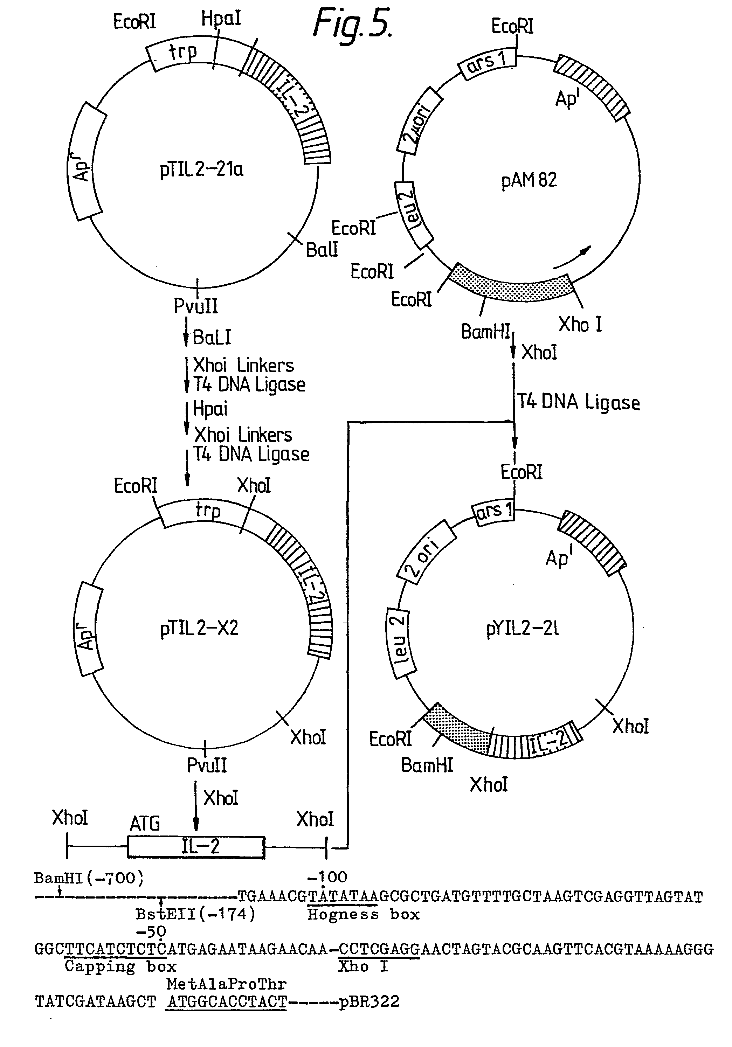

Figure 5 is a flow chart showing the construction
of a recombinant DNA (pYIL 2-21) using paM82 as a
vector.

In the Figures, "A", "G", "C" and "T" represent
deoxyadenylic acid, deoxyguanylic acid, deoxycytidylic
acid and thymidylic acid, respectively.

BRIEF DESCRIPTION OF THE PREFERRED EMBODIMENT

The cloned gene, coded for an IL-2 polypeptide,
may be obtained by transcription of messenger RNA
(mRNA; "RNA" is an abbreviation for ribonucleic
acid) corresponding to IL-2 (hereinafter referred to
as "IL-2 mRNA") and originating from a mammalian cell
which is characterized by the capability of producing
a polypeptide which possesses IL-2 activity, as a
complementary DNA (cDNA). The single stranded cDNA
(ss-cDNA) obtained can be converted into a double
stranded cDNA (ds-cDNA).

The mRNA used as a template for the preparation of
cDNA can be conventionally separated from a mammalian
cell capable of producing IL-2 polypeptide. The
separated RNA is polyadenylated (Gillis et al.,
Immunological Rew., 63, 167-209 (1982)), and the polyadenylated RNA can be fractionated by, for example, centrifugation on a sucrose density gradient as a sediment of 11 to 12S. Occasionally mRNA of 13S will demonstrate IL-2 mRNA activity, and in those instances, it is presumed that the mRNA is in an aggregated form of 11 to 12 S mRNA.

The mammalian cells capable of producing IL-2 which are the source of mRNA of the present invention, may be T-lymphocytes, such as peripheral blood mononuclear cells, tonsil cells, spleen cells or the like, which are operationally obtainable from mammals. The cells may be conventionally pretreated such as with a nylon column, antiserum-complement, density gradient fractionation, multiple enzyme treatment such as a combination of neuraminidase and galactose oxidase, by x-ray irradiation or with trypsin to confer the cells with the IL-2 productivity or to increase the I L-2 activity. Also cloned T lymphocytes, obtained from the said mammilian cells after cultivation in the presence of T cell growth factor, may be also used as a source of mRNA and is the preferred T-lymphocytes. Transformed lymphocyte cell lines such as T lymphocytes derived from leukemia or lymphoma cell line per se or from their derivatives obtained by pretreatment or mutation by the methods mentioned above, or the cloned transformed cell lines are preferred as sources of the mRNA. Evidently, cloned cells line usually contain larger amounts of IL-2 mRNA as compared to parental bulk cell lines. T cell hybridomas, obtained by fusion of the lymphocyte derived cells mentioned above and tumor cell lines, such as CEM, Molt 4F, and BW5147, are also preferred mammalian cell lines for use in this invention. In such instance the lymphocyte derived cell lines include (1) constitutive producers of IL-2 and (2) those which are producers of IL-2 only in the presence of a mitogen introduced into the culture, either in the absence or presence of other IL-2 production co-stimulatory cells.

In order to generate IL-2 mRNA in constitutive IL-2 producer cells, the constitutive IL-2 producer cells are cultured under conditions commonly known in the field of cell culture. For the generation of the mRNA in cells producing IL-2 only in the presence of mitogen, cultured cells are washed extensively with culture medium and resuspended in a culture medium, such as Rosewell Park Memorial Institute 1640 (hereinafter "RPMI 1640"), Dulbecco Modified Eagle Medium (hereinafter "DMEM") or in Click's medium, which may or may not contain serum.

These culture media may be supplemented with various additives, such as penicillin, streptomycin or other antibiotics, or with fresh L-glutamine, Hepes buffer and sodium bicarbonate in a concentrations as are generally used in the field of cell culture. The preferred cell density may be from 0.5 to 4 x $10^6$ cells/ml. To induce activation of the mRNA and production of IL-2, appropriate stimulants are added. Suitable such stimulants include mitogens, neuraminidase, galactose oxidase, zinc derivatives such as zinc chloride, or lymphocyte activating substances originated from microorganisms, such as protein A, streptolysin-O. The stimulated cells are recovered and washed. The co-presence of macrophages or dendritic cells during the mitogen stimulation may also activate the mRNA, or may increase the amount of the activiated mRNA. Likewise the co-presence of cell lines derived from B lymphocytes or B lymphocyte lines, such as Raji, Daudi, K562, and BALL-1 may activate the mRNA or increase the amount of activated mRNA.

To propagate the mammalian cells, they are maintained in an _in vitro_ cell culture or in histocompatibility matched animals, under normal conditions. When _in vitro_ culture maintenace is used to prepare the source of mRNA, the cells may be grown in any of the culturing media as were previously found to foster growth of T cells. These culture media may or may not be supplemented with mammal serum, serum component or serum albumin.

The culture period for the activation of the mRNA will correspond to the period necessary for the activation of cells to generate the mRNA. This period usually coincides with the time needed to start the excretion of IL-2 into the culture medium. The preferred period may be from 3 to 12 hours after addition of a stimulant, such as a mitogen. Undue prolongation of the culture period may occasionally result in the decomposition of the generated IL-2 mRNA. During the course of the activation of IL-2 producing cells, phorbol esters, such as PMA or TPA may preferably utilized in a concentration from 10 to 50 ng/ml to boost the level of activation.

The above described process for activation of IL-2 mRNA may be carried out at temperatures ranging from 32 to 38°C in a humidified atmosphere and in a pH of approximately 7.0 to 7.4.

The procedures to obtain and culture mammalian cells capable of producing IL-2 will now be explained.

(1) Acquisition of constitutively IL-2 producing cell line.

Jurkat cell line of human leumemic T cell (freely available from Fred Hutchinson Cancer Institute, Seattle, United States, Salk Institute, San Diego, United States, German Cancer Centre, Heidelberg, West Germany) is suspended in Click's medium at a cell density of $1 \times 10^6$ cells/ml and $8 \times 10^3$ R x-ray is irradiated at irradiation rate of 150 R/min. Thereafter 0.1 cells of the thus irradiated cells per 200μl of medium are inoculated into Click's medium containing 5% FCS in 96 well flat-bottom microplates (Falcon 3072) and cultured for 3 weeks at 37°C in 5% $CO_2$ incubator (cloning by limiting dilution method). The grown viable

0142268

cells are transferred into 24 well culture plate (Nunc) before the cell layer becomes confluent and is further cultures for 5 days. The grown cells are further cultured in serum and serum albumin free synthetic culture medium for about two days at an initial cell density of between 1-2 x $10^6$/ml. The culture supernatant is harvested by centrigugation, and filtered with 0.22 millipore filter paper to clear off debris and to sterilize the supernatant, and then x-ray treated mutants capable of producing IL-2 constitutively are selected and cloned by measuring the IL-2 activity present in the supernatant.

(2) Acquisition of IL-2 producer cell from human peripheral blood mononuclear cells.

Human peripheral blood is harvested and peripheral blood lymphocytes (hereinafter "PBL") are isolated by density gradient centriguation on Ficoll-Hypaque. The PBL is inoculated in 2 ml of Click's medium containing 5% FCS at a cell density of 1 x $10^6$ cells/ml in 24 well Nunc culture plate together with 100µl of 5µg/ml of phytohemmaglutinin-M(Gibco) (pHA), and cultured for 48 hours under the conditions described above. The cells are washed and inoculated again in 1 ml of Click's medium at a cell density of 1 x $10^5$ cells/ml together with 1 ml of a conditioned medium which has been prepared from human splenocytes stimulated by 2.5µg/ml of concanavalin A (hereinafter "Con A") for 48 hours, and the culture medium containing 50% conditioned medium is exchanged in every three days to get long term culture of human T lymphocytes from PBL. The thus prepared long term cultured human T lymphocytes are cloned by the limiting dilution method as described above, in the presence of human splenocytes derived conditioned medium and the cell clones are propagated similarly. Thereafter cloned human T lymphocytes are inoculated in 1 ml of RPMI 1640 at a cell density of 1 x $10^6$ cells/ml in 24 well Nunc culture plate in the presence of 10µg/ml of PHA and cultured for 24 hours at 37$^O$C in 7.5% $CO_2$ incubator. The supernatants of the culture liquid are harvested, centrifuged, filtered through a 0.22μ millipore filter and assayed for IL-2 activity to specify the IL-2 producing human normal T lymphocytes clones.

(3) Acquisition of malignant cell line derived from human lymphocytes capable of producing IL-2 in the presence of mitogen.

Jurkat cell line or cloned cell lines such as Jurkat 111 obtained by the limiting dilution method described above are able to produce from 10 to 4,000 units/ml of IL-2 when cultured for 24 hours in a serum free synthetic medium described previously or in RPMI 1640 containing 1-2 % mammalian serum in the presence of a mitogen such as 10μg/ml Con A or 2.5μg/ml PHA. These malignant human cell lines also produce IL-2 when cultured in the presence of zinc chloride, protein A or picibanil.

(4) Acquisition of cells capable of producing Il-2 in the co-presence of a mitogen and other co-stimulatory cells or co-stimulatory soluble factors.

Human malignant cell line Molt 4F and some cloned cell lines such as Jurkat J99, obtained according to the limiting dilution method, do not produce IL-2 even when cultured for 24 to 72 hours in the presence of lectins or mitogens in any concentration. However, these cells become able to produce IL-2 in significant amount (10-100μ/ml) during culture period of 24 hours at 37$^O$C , when cocultured with 5-10μ/ml interleukin 1, one of monokines, or with 50% number of K562 or Raji cells.

The extraction of IL-2 mRNA from cells activated by the manner as mentioned above is carried out according to the conventional well known procedures, irrespective of the difference of cell sources. For instance, cells are partially or completely disrupted by additon of a detergent such as NP-40, SDS, Triton-X and deoxycholic acid or by mechanical homogenization

-9-

or freeze-thawing. To prevent degradation of RNA by ribonuclease during extraction of mRNA, it is preferred to add RNase inhibitors such as heparin, polyvinyl-sulfate, bentonite, macaroid, diethylpyrocarbonate or vanadyl complex. IL-2 mRNA can be obtained from precipitated polysome in the IL-2 biosyntheis, which is precipitated with anti-IL-2 antibody by extracting with a detergent.

The poly A-containing mRNA can be fractionated or concentrated by an conventional manner, such as by affinity chromatograph or batch absorption on oligo dT-cellulose, poly U-sepharose of sepharose 2B, sucrose density gradient centrifugation or by agarose gel electrophoresis.

The mRNA fractions are then assayed for IL-2 mRNA activity by testing biological activities of proteins translated from the mRNA fractions or by identifying the translated protein using monoclonal antibody against the IL-2 peptide. For instance mRNA is usually translated into the corresponding protein by microinjection into frog (Xenopus laevis) egg (Gurdon, J.B., et al., Nature, 233 , 177-182 (1972)) or by employing the mRNA dependent reticulolysate or wheat germ translation cell free systems.

The activity of IL-2 may be ascertained by the microassay procedure principally discussed by Gillis et al (Gillis. S., et al., J. Immunol., 120, 2027-2033 (1978)). The assay monitors the IL-2 dependent cellular proliferation of a cytotoxic T lymphocyte cell lines (hereinafter "CTLL") generated according to the methods described by Gillis et al., That is, $4 \times 10^3$ CTLL cells are inoculated into 100μl of RPM1 1640 medium containing 2% FCS in 96 well flat-bottomed microplates together with 100μl of the serially diluted translation products. After 20 hours incubation at $37^{\circ}C$ in 5% $CO_2$ incubator, cells are pulsed for 4 hours with 0.5 μCi of $^3$H-TdR, harvested onto glass fibre strips with the aid of an automated cell harvester and then the incorporated radioactivity is measured by liquid scintillation counting.  By these assay procedures, the CTLL cells cultured in the presence of IL-2 were found to incorporate $^3$H-TdR in a dose dependent manner resulting in the definite calculation of the amount of IL-2 contained in test samples.

IL-2 possesses the activity to promote the proliferation of T lymphocytes, which enables the measurement of IL-2 activity using an index of T cell growth activity.  That is, five CTLL cells are transferred into 100μl of DMEM containing 2% FCS in 96 well flat-bottomed microplates together with 100μl of the serially diluted translation products. After 72 to 96 hours incubation at 37$^O$C in a 5% $CO_2$ incubator, the number of cells grown and activated is counted under microscopy.  As an positive external control group, 100 units/ml, 10 units/ml of IL-2 are added and the IL-2 activity of the test sample is calculated in comparison with the number of grown viable cells in these control groups.

The thus obtained IL-2 mRNA from the most active fraction is used as a template to synthesize ds-cDNA and the ds-cDNA is connected with a vector DNA. Synthesis of cDNA is carried out by conventional procedures.

At first, ss-cDNA which is complementary to mRNA is prepared in the presence of dATP, dGTP, dCTP, dTTP employing reverse transcriptase and using mRNA as a template and oligo-dT as a  primer.  The template mRNA is then removed by alkaline treatment and ds-cDNA is achieved by employing reverse transcriptase or DNA polymerase and using the above synthesized ss-CDNA as a template.

Recombinant DNA is prepared from the ds-cDNA thus obtained and a vector DNA containing replicon capable of replicating in cells of Saccharomyces cerevisiea .  The recombinant DNA is  thereafter incorporated into the host cells.

The ds-cDNA and the vector DNA capable of propagating in cells of Saccharomyces cerevisiae are, prior to ligation, modified by various procedures such as exonuclease treatment, additon of chemically synthesized DNA pieces and G, C-tailing to give ligatable termini to the ends of the ds-cDNA and the vector DNA. Ligation of the ligatable DNAs is performed by, for example, $T_4$-phage DNA ligase in the presence of ATP.

With the recombinant DNA thus obtained, living cells are transformed to amplify the cloned cDNA or to produce IL-2 polypeptide.

The ds-cDNA can be combined with a vector capable of propagating in Escherichia coli or both in Escherichia coli and Saccharomyces cerevisiae, by which gene coding for IL-2 polypeptide can be selected using Escherischia coli. The selected gene is thereafter inserted into a vector capable of propagating in Saccharomyces cerevisiae, if the vector used for the selection was that capable of propagating only in Escherichia coli.

Examples of vectors capable of propagatin in Escherichia coli are pJDP219, pJDP218, YRp3, YEp13, YIP1, pYC1, pYC2, YEp213, YRp17, YIp203 and pPGK.

Transformation of the host cell with the recombinant DNA may be carried out efficiently after forming a protoplast of the host cell.

Cells possessing IL-2 gene can be isolated after the transformation, by either of the following two ways.

(1) In the plus-minus method, partially purified IL-2 mRNA is obtained by sucrose density gradient centrifugation of mRNAs extracted from mitogen activated mammalian cells as 11 to 12s sediment and then 32p-radiolabelled ss-cDNA is synthesized using the partially purified mRNA as a template. After removal of the template mRNA by alkaline treatment, isolated cDNA is hybridized with partially purified 11 to 12s mRNA extracted from mitogen non activated mammalian cells. Thereafter nonhybridized and hybrid forming cDNA are fractionated on hydroxylapatine column chromatography. The non hybridized cDNA and hybridized cDNA are tentatively called probe A and probe B, respectively. Transformants are grown on two nitrocellulose filters in quite the same way: and the DNA of the cells is fixed on the filter paper by alkaline treatment. Probe A and Probe B are respectively hybridized with the DNA on two different filter papers and thereafter autoradiography assay is carried out to select the transformants which react positively to probe A (plus), but react weakly or do not at all to probe B (minus) Taniguchi et al., Proc. Jpn. Acad., v 155B 464-469, 1979).

(2) The second method consists of dividing, for example, 1,000 to 10,000 transformant clones into several tens or several hundreds of clone groups. The divided clone groups are respectively cultured by conventional means to obtain plasmid DNAs. Thereafter these plasmid DNAs are conv⌐erted into ss-cDNAs, for example, by heat denaturation, and the ss-cDNAs obtained are fixed onto nitrocellulose filter papers to achieve the hybridization of mRNA complementary to the fixed DNAs and prepared from mammalian cells including activated Il-2 mRNA. Alternatively, mRNAs containing IL-2 mRNA are hybridized with heat denatured plasmid DNAs and the DNA-mRNA hybrid is fixed onto nitrocellulose filter papers. These filter papers are then washed with low salt concentration buffer, such as 1mM HEPES, or with 10mM NaCl, and mRNA adsorbed on filter paper is extracted by treatment with a solution containing 0.5mM EDTA and 0.1% SDS solution for e.g. 1 min. at 95$^{\circ}$C. Purified mRNA is recovered by elution through oligo dT-cellulose colum chromatography. Thereafter, the mRNA is translated into protein by microinjection into <u>Xenopus</u> <u>laevis</u> egg to ascertain IL-2 activity, or the mRNA is translated into a protein using the mRNA dependent reticulocyte or wheat germ <u>in</u> <u>vitro</u> cell free translation system, to analyse IL-2 activity using anti-IL-2 antibody. According to these procedures, the group in which the presence of IL-2 activity was detected was further divided repeatedly into groups consisting of smaller number of transformant clones until a single clone possessing IL-2 DNA is specified.

To obtain cDNA coding for IL-2 polypeptide from the IL-2 producing transformant, the recombinant DNA in the transformant is separated and cleaved with a restriction endonuclease. From the DNA fragments formed by the cleaving, the insert cDNA fraction is separated.

The complete nucleotide sequence of the PstI DNA insert coding for IL-2 polypeptides from the recombinant DNA of pIL2-50A was determined by the procedure of Maxam and Gilber (Meth. Enzyme. <u>65</u> 499-560, (1980)) and by the dideoxynucleotide chain termination method (Smith, A.J.M. Meth. Enzym. <u>65</u>, 560-580 (1980)).

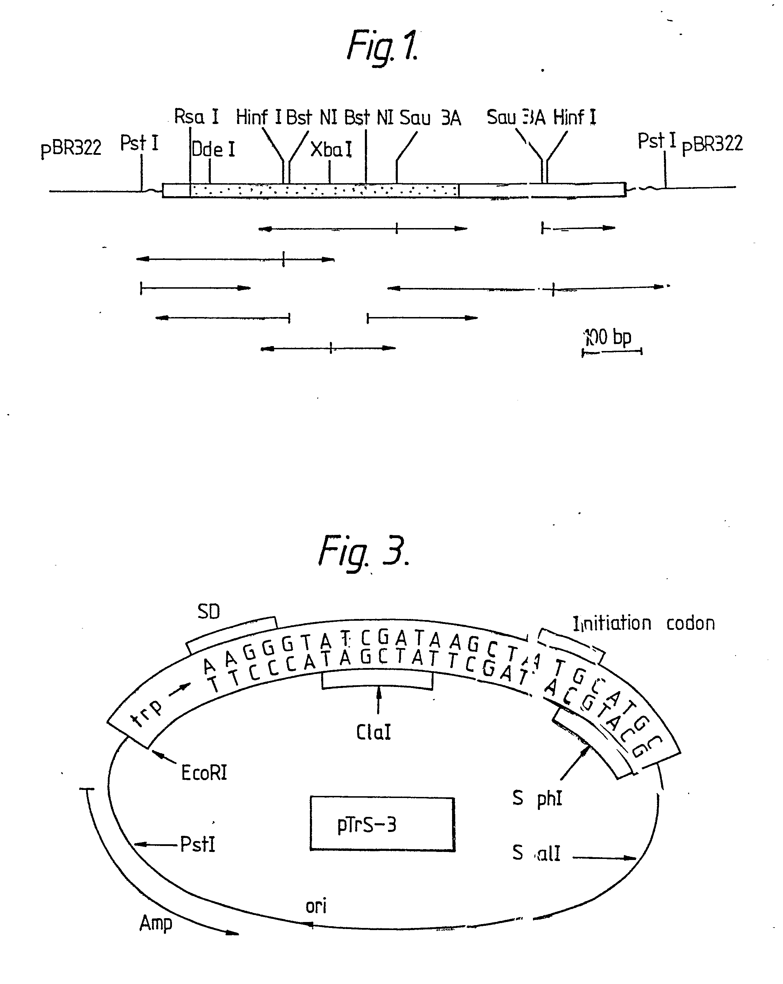

The restriction endonuclease cleavage map of the cDNA insert and base sequence of the insert are shown in Figure 1, and Figure 2(2) in which the cDNA has sites cleaved with restriction endonuclease of BstNI, XbaI and BstNI in this order, respectively.

The DNA sequence of the insert contains a single large open reading frame. The first ATG sequence, which usually served as the initiation sequence in eukaryotes (Kozak, M. Cell, <u>15</u>, 1109-1123 (1978)), is found at nucleotides 48-50 from the 5' end. This ATG is followed by 152 codons before the termination triplet TGA is encountered at nucleotides 507 to 509.

A stretch of A residues corresponding to the 3'-poly (A) terminus of the mRNA is found at the end of the cDNA and this is preceeded by the hexanucleotide AATAAA (position 771-776) which is usually found in most eukaryotic mRNAs (Proudfoot, N.J. and Brownlee, C.G., Nature 263, 211-214, (1976))

The amino acid sequence, for which the cDNA codes, could be deduced as shown in Figure 2(b) (Amino Acid sequence I), and the polypeptide of the amino acid sequence I consists of 153 amino acids and its molecular weight is calculated to be 17631.7 daltons. As has been reported as a common feature in most of the secretion proteins known to date (Blobel, G. et al., Sym. Soc. exp. Med., 33, 9-36 (1979)), the N-terminal region of the deduced IL-2 polypeptide is also quite hydrophobic and this region probably serves as a signal peptide which is cleaved during the secretion process of the mature IL-2. Such cleavage occurrs either between Ser and Ala at position 20 and 21 or between Ala and Pro at position 21 and 22 respectively, forming the polypeptide having amino acid sequences II and III, since similar cleavage sites have often been found in other secretion proteins (Blobel, G. et al., Symp. Soc, exp. Med. 33, 9-36, (1979)). The mature IL-2 polypeptide would then contain 133 or 132 amino acids with the calculated molecular weight being 15420.5 daltons or 15349.4 daltons. This value is then compared with the reported value for human IL-2 protein from Jurkat cells (15,000 daltons)(Gillis,S. et al., Immurological Rev., 63, 67-209, (1982)). Additionally, the DNA fragment initiating from CCT codon at position 111 to 113 in base sequence, which, therefore, codes for polypeptide initiating from Pro at position 22 (Amino Acid Sequence III in Figure 2 (b)), was confirmed to express a polypeptide possessing IL-2 activity as shown in Example 5. It is also confirmed that the DNA fragment initiating from GCA sequence at position 107 to 110 in the base sequence, which therefore codes for a polypeptide initiating from Ala at position 21 (Amino Acid

0142268

Sequence II in Figure 2 (b)) expresses a polypeptide possessing IL-2 activity as shown in Example 8.

It has been known that genes of eukaryotes often show polymorphysm for example in human interferon genes. (Taniguchi et al. Gene 10 11-15 (1980), Ohno & Taniguchi, Proc. Natl. Acad. Sci USA, 77, 5305-5309, (1986); Gray et al., Nature 295 501-508 (1981)). In some cases, polymorphysm is accompanied with replacement of certain amino acids of the protein products and in other cases, the structure of the protein product remains unchanged. In the case of human IL-2 cDNA, another cDNA clone (pIL2-50A) in which the A residue at position 503 of pIL2-50A cDNA (Fig 2) is replaced by a G residue can be detected. Other cDNA clones with some base substitution compared to pIL 2-50A cDNA can also be expected.

As can be understood from the above the genes of present invention include DNA having the base sequence shown in Fig 2(a), DNAs initiating from ATG sequence at position 48 to 50 and having the sequencial bases following the ATG sequence up to at least ATC sequence at position 504-506, DNAs initiating from GCA sequence at position 108-110 and having the sequencial bases following the GCA sequence up to at least the ATC codon and DNAs initiating from CCT sequence at position 111-113 and having the sequencial bases following the CCT sequence up to at least the ACT sequence. The genes of the present invention also include DNAs ending at the ACT sequence at position 504 to 506 and initiating from A at position 1, ATG sequence at position 48 to 50, CGA sequence at position 108 to 110 or CCT sequence at position 111 to 113. The genes of the present invention further include DNAs ending at TGA sequence at position 507 to 509 and initiating from A at position 1, ATG sequence at position 48 to 50, GCA sequence at position 108 to 110 or CCT sequence at position 111 to 113. The genes of the present invention furhter include DNAs ending at C at position 801 and initiating from A at position 1, ATG sequence at position 48 to 50, GCA sequence at position 108 to 110 or CCT sequence at position 111 to 113. The genes of the present invention additionally include DNAs ending with poly (A) and initiating from ATG codon at position 48-50, GCA sequence at position 108-110 or CCT sequence at position 111 to 113. The genes of the present invention also include those of which base sequence correspond to Amino Acid Sequence I, II and III. Furthermore, polypeptides deficient in one or more amino acids in Amino Acid Sequence I, or polypeptides in which one or more amino acids in Amino Acid Sequence I are replaced with one or more amino acids may have IL-2 activity. Therefore genes coded for such polypeptides are suitable genes for the present invention. Similarly, genes having additive connection of one or more base sequences, capable of expressing one or more amino acids to Amino Acid Sequences I, II or III, are suitable in this invention so far as the additively connected amino acids do not interfere with the action of the polypeptides in expressing IL-2 activity. Modified additively connected amino acid region which interfere with the polypeptide function as IL-2, can be used in this invention so far as the additively connected region can be easily eliminated. This situation is quite the same for the additive connection of DNA to the 3'-terminus of genes corresponding to Amino Acid Sequence I, II and III coding additional amino acids at C-terminal of the I, II and III having Amino Acid Sequence I, II and III respectively. Therefore use of genes coded for such polypeptides are to be considered to be included in the present invention.

Recominant DNAs which direct the production of IL-2 in living cells can be constructed by various methods.  For example, the coding sequence of IL-2 cDNA can be inserted in an expression vehicle at the position downstream of the promoter sequence. Alternatively, a cDNA piece carrying a promoter sequence can be inserted upstream of the IL-2 coding sequence, after or prior to, the insertion of cDNA in the expression vehicle.

Example for procedures to construct Saccharomyces cerevisiae which expresses the IL-2 cDNA and produce IL-2 polypeptide is explained more precisely below:

A yeast-E. coli shuttle vectors pAT77 and pAM82 have been described by Miyanoshita et al.  (Proc. Natl. Acad. Sci. USA 80, 1-5. (1983)).  The vector pAM82 is a derivative of pAT77 and both carrying markers of ars 1 (Stinchcomb, D.T. et al, Nature 282, 39-43, (1979), 2μm ori (Broach, J.R. et al. Gene 8, 121-133 (1979)) and leu 2 (Ratzkin, B. et al. Proc Natl. Acad Sci USA 74, 474-491 (1979)) and the promoter for the yeast acid phosphatase (APase) gene.  They also carry a 3.7 kb DNA segment of pBR322 which contains an ampicillin resistance marker (AP$^\gamma$) and the origin of replication (Fig. 5).  The APase promoter is inducible by shifting a high concentration of phosphate into a low concentration in the culture media.  In order to express human IL-2 cDNA, pIL2-50A is digested by PstI and after treating either by the E. coli Klenow Fragment of by T4 DNA polymerase, the cDNA is ligated with pAM82 previously digested by XhoI and incubated with the E. coli Klenow Fragment to fill in the ends. Hybrid plasmids in which the cDNA coding sequence are downstream of the yeast APase promoter sequence are selected by cloning them in E.coli.  The obtained plasmid, pYIL-2A, is introduced into yeast and, after induction of the APase promoter, IL-2 activity in the yeat extract is measured.  The plasmid pYIL-2a contains a stretch of GC residues between the yeast promoter and the IL-2 cDNA.  It is possible that such a sequence inhibits the expression of IL-2 cDNA.  In order to overcome this problem following construction of a plasmid can be made:  Plasmid pIL2-50A is digested by PstI and the cDNA inserted is isolated.  This cDNA is then treated by T4 DNA polymerase in the presence of dATP, dGTP and dTTP so that the stretches of C-residues at the both ends of the cDNA are chewed off and subsequently treated by Nuclease S1, to remove stretches of G-residues.  This DNA is ligated with XhoI DNA linker and plasmid pBR322 whose EcoRI site is cleaved and rendered flush by EcoRI and the Klenow Fragment, and the resulting plasmid, pIL2-Xho, is digested by XhoI and the cDNA inserted is isolated. The cDNA is then introduced into the single XhoI site of pAM82 and a plasmid containing the IL-2 coding sequence correctly oriented with respect to the yeast APase promoter is cloned in E. coli.  The plasmid, pYIL-2B, is introduced into yeast and, after induction of the APase promoter, IL-2 activity in the yeast extract is measured.

Cells incorporating the recombinant DNA are cultured to amplify the recombinant DNA or to produce IL-2 polypeptide.  The cultivation is carried out by conventional means.  For instance, transformed yeast may be cultured in a medium containing source of carbon, a nitrogen source, inorganic salts and, when required, organic nutrients such as vitamin and amino acid at a temperature in the range from $20^{\circ}$ to $37^{\circ}$C and a pH ranging from 4 to 7 under aerobic condition.

The IL-2 produced intracellulary or extracellulary is recovered by any known method, such as precipitation with ammonium sulfate, dialysis to remove salts (under normal or vacuum pressure), gel filtration, chromatography, preparative flat-bed isoelectric focusing, gel electropheresis, high performance liquid chromatography (hereinafter "HPLC"), (ion exchange, gel filtration and reverse phase chromatography), and affinity chromatography on dye bound carrier, on activated Sepharose 4B coupled with monoclonal antibody against said IL-2 or on lectin bound Sepharose 4B and the like. Methods of recovery, the purification of IL-2, are described in Watson et. al., J. Exp. Med., <u>150</u>, 849-861 (1979), Gillis et al., J. Immunol., <u>124</u>, 1954-1962, (1980), Mochizuki et. al., J. Immunol Methods <u>39</u>, 185-201, (1980), and Welte, K. et al., J. Exp. Med., <u>156</u>, 454-464 (1982).

The polypeptide thus obtained shows the same biochemical and biological behavior as has been known for IL-2 produced by mammalian cells by mitogen stimulation, and has IL-2 activity. The molecular weight is around 15,000 dalton and IL-2 activity was completely neutralized or precipitated with monoclonal anti-IL-2 antibody in the presence or absence of immunoabsorbents, such as Igsorb (Enzyme Centre). In immunoelectrophoresis, the IL-2 polypeptide shows only a single precipitate against the corresponding anti-IL-2 antibody. The IL-2 activity remains stable after reduction with 2-mercaptoethanol, and is resistant to treatment with DNAse and RNAse as well as to heat treatment at 56°C for 30 min. The activity is stable at a pH between pH 2 to 9. The IL-2 produced could promote the growth of monoclonal functional T cells (cytotoxic T lymphocyte), enhance the thymocyte mitogenesis, give rise to the generation of anti-tumor specific cytotoxic T lymphocytes from memory state in the absence of the antigen, and could be used to augment natural killer cell activity against YAC-1 and RL$\hat{0}$1 cells.

Having now generally described this invention, the same will become better understood by reference to certain specific examples which are included herein for the purpose of illustration only and are not intended to be limiting unless otherwise specified.

Example

A, Preparation of gene coding for IL-2 polypeptide-(1)

(1)  Human T leukemia cell line, Jurkat cells (freely available in Japan, W. Germany and United States) were suspended in RPMI 1640 medium containing 10 vol/vol % FCS and were irradiated with x-ray till 10,000 roentgen at a room temperature for 50 seconds using x-ray irradiation apparatus Exc 150/300 - 4 (Toshiba, Japan), and thereafter the irradiated cell was cultured for 5 days at 37$^O$C in 5% $CO_2$ incubator at a initial cell density of 1 x 10$^5$ cells/ml in the culture medium mentioned above.  The mutated cells (0.2 cells/well) were placed in wells 10 pieces of flat-bottomed microplates having 96 wells, and cultured at 37$^O$C in 5% $CO_2$ incubator for 21 days.  Clones obtained from the wells showing growth were repeatedly transferred into fresh culture medium to propagate the clone sizes, and the propagated clones were cultured for 24 hours at an initial cell density of 1 x 10$^6$ cells/ml in the presence of 50 µg/ml of Con A and IL-2 activity was measured according to the methods described before.  Consequently a human T cell line designated as Jurkat-111 (hereinafter "J-111")  (ATCC CRL 8129), cloned from parent Jurkat, was selected, of which productivity of IL-2 was increased 40 times as much as that of the parent strain.  The cloned cell line J-111 could grow under conventional conditions and the growth rate shows almost the same with ordinary Jurkat cells.

(2)  Cells (1 x 10$^5$/ml) of J-111 were inoculated in 1,000 ml of serum free synthetic culture medium RITC 55-9 (Sato, T.  et al., Exp. Cell Res., 138, 127-134 (1982)) in roller culture bottles (Falcon 3037) and cultured for 4 days at 37$^O$C, and cells propagated were harvested by centrifugation.  The harvested cells were again inoculated in the medium mentioned above which had been added with 25 µg/ml of Con A to contain 4 x 10$^6$ cells/ml.  In four batches of roller culture bottles (Falcon), 1,000 ml of the inoculated culture medium was placed into each batch. The cultivation was continued for 6 hours with rotating.

(3) Jurkat cells ($1.2 \times 10^6$) thus stimulated with 25 µg/ml of Con A for 6 hrs were suspended in 8,000 ml of phosphate buffer balanced with saline (hereinafter "PBS"). The cells were washed twice by centrifugation and were resuspended in 800 ml of RSB solution (10 mM Tris-HCl, pH 7.5, 10 mM NaCl, 1.5 mM $MgCl_2$) containing Ribonucleosides-Vanadyl Complex (10 mM), an inhibitor of nuclease. Then a detergent NP-40 was added to contain 0.05% as final concentration, followed by gentle mixing and the cell nuclei were removed by centrifugation for five minutes at 3,000 rpm at $4^{\circ}$C. SDS (0.5%) and EDTA (5 mM) were added to the supernatant and cytoplasmic RNA was extracted by addition of equal volume of phenol. After three times extraction with phenol, RNA was precipitated with two times volume of ethanol and precipitates were collected by centrifugation, which were solubilized in 10 mM Tris-HCl of pH 7.5. The amount of RNA obtained was 196mg.

Fractionation of mRNA was carried out using affinity chromatography on oligo (dT)-Cellulose (P.L. Biochemicals, Type 7). An adsorption solution was a solution of pH 7.5 containing 20 mM Tris-HCl, 0.5 M NaCl, 1 mM EDTA and 0.5% SDS and elution was carried out with water and 10 mM Tris-HCl (pH 7.5) by turns after washing the colum with the buffer (20 mM Tris-HCl, pH 7.5, 0.5 M NaCl, 1 mM EDTA). The resultant mRNA eluted was 3.6 mg. Next, 2.4 mg of the mRNA obtained was fractionated by sucrose density gradient centrifugation (5 to 2.5% sucrose density gradient in a solution of pH 7.5 containing 50 mM Tris-HCl, 1mM EDTA and 0.2 M NaCl, centrifuged at 26,000 rpm for 24 hours at $4^{\circ}$C), and 11 to 12S fraction of mRNA was fractionated into fractions No. 12, 13, 14 in the amount of 59 µg, 46 µg and 60 µg, respectively.

(4)   The mRNA obtained in fraction No. 13 was microinjected into the cocyte of <u>Xenopus</u> <u>laevis</u> (50 ng mRNA/egg) and the culture supernatant was served for the assay of IL-2 activity.  As shown in Table 1, the increase of the incorporation of $^3$H-TdR and the increase of number of activated T lymphocytes were confirmed, clearly verifying that mRNA in this fraction contains human IL-2 mRNA.·

(5)   Thereafter cDNA was synthesized <u>in</u> <u>vitro</u> from No. 13 fraction of 11 to 12S mRNA containing IL-2 mRNA and recombinant DNA was constructed with the plasmid vector PBR 322.  With the  recombinant DNA, <u>Escherichia</u> <u>coli</u> was transformed, and clone acquired IL-2 cDNA clones was selected, as follows:

(5-1)  Fifty mM Tris-HCl buffer (pH 7.5), 30 mM NaCl, 6 mM MgCl$_2$, 5 mM dithiothreitol (hereinafter "DTT"), 0.5 mM of each dATP, dGTP, dCTP, dTTP (dCTP contained $^{32}$p radiolabelled one), 0.7 µg oligo (dT)$_{10}$, 10 µg mRNA and 15 unit AMV reverse transcriptidase (J. W. Beard) were mixed and maintained for 90 min. at 41$^O$C.  After termination of the reaction, DNA was recovered as  ethanol precipitates after the phenol treatment, and DNA was solubilized in a solution of pH 7.5 containing 20 mM Tris and 1 mM EDTA. Two point five µg of ss-cDNA was synthesized.  To remove mRNA present in this solution, the solution was made 0.33 N-NaOH by additon of NaOH, allowed to stand for 15 hrs at a room temperature, then the solution was neutralized with equal volume of 1 m-Tris-Hcl of pH 7.5 and passed through "Sephadex G-50" column. The recovered cDNA was 1.8 µg.

(5-2)  Fifty mM phosphate buffer (pH 7.5), 10 mM MgCl$_2$, 10 mM DTT, 0.75 mM of each dATP, dGTP, dCTP, dTTP (dCTP contains $^3$H radiolabelled one) , 1.8 µg ss-cDNA, and 8 unit of polymerase I (BRL, United States) were mixed and were allowed to react for 15 hours at 15$^O$C.  After the termination of the reaction, DNA was recovered as ethanol precipitate, after treatments with phenol and with chloroform. 1.10 μg of ds-cDNA was generated. A mixture of 50 mM sodium acetate (pH 4.5) 0.2 M Nacl, 1 mM $ZnCl_2$ and 1.10 μg of ds-cDNA was incubated for 20 min. at 37°C, added with 0.25 unit of nuclease $S_1$ (Sankyo, Japan), and incubated further for 15 min.

After the termination of the reaction, the reaction product treated twice with phenol was applied onto sephadex G-50 to get 0.55 μg of ds-cDNA.

(5-3) A mixture of 0.14 M potassium cabodylate, 30 mM Tris base, 0.1 mM DTT, 1 mM $COCl_2$, 0.64 mM $^{32}$p-dCTP (spc. act. $2.7 \times 10^6$ cpm/n mol), 0.55 μg of ds-cDNA and 5 unit of terminal transferase (BRL) were incubated for 7 min. at 37°C, then applied onto sephadex G-50 colum after phenol treatment to get 0.50 μg DNA as ethanol precipitates. The recovered DNA was found to be extended with around 50 dCMP residues at the both 3' terminus.

Ten μg of pBR 322 DNA was cleaved with restriction enzyme PstI, and 3'-termini of the cleaved DNA were added with dGMP chain, by the same method as that used in the addition of dCMP to ds-cDNA mentioned above, except dGTP was used in place of dCTP.

(5-4) A mixture of 50 mM Tris-HCl (pH 7.5), 0.1 M NaCl, 5 mM EDTA, 0.05 μg of pBR 322 elongated with dGMP residues and 0.01 μg of cDNA extended with dCMP was incubated firstly for 2 min. at 65°C, then for 120 min. at 46°C, for 60 min. at 37°C and finally for 60 min. at a room temperature. E. coli x 1776 (Curtiss III, R. et al., in Molecular Cloning o Recombinant DNA, (W.A. Scott & R. Werner ed).) Academic Press, (1977)) was inoculated in 50 ml of L broth containing 100 μg/ml of diaminopimelic acid, 50 μg/ml of thymidine, 1% trypophan, 0.5% yeast extract, 0.5% NaCl and 0.1% glucose and cultured in shaking at 37°C until the absorbance of culture liquid at 562 nm became around 0. D 0.3. After the termination of the culture, the culture liquid was left at 0°C for 30min., then the bacterial cells were collected by cenrifugation followed by twice washing with 25ml of a solution containing 5 mM Tris-HCl (pH 7.6), 0.1 M NaCl, 5 mM MgCl$_2$ and 10 mM RbCl.

Thus obtained cells were suspended in 20 ml of a solution containing 5 mM Tris-Hcl (pH 7.6), 0.25 M KCl, 5 mM MgCl$_2$, 0.1 M CaCl$_2$ and 10 mM RbCl and were left at 0°C for 25 min., then the cells were collected to resuspend them into 1 ml of the same solution, the recombinant DNA described above was added into 0.2 ml of the cell suspension and the suspension was left at 0°C for 60 min. Then 0.7 ml of L broth was added to culture in shaking for 30 min. at 37°C. Thus obtained culture medium (0.1ml) was thoroughly spread on the surface of 1.5% agarose medium composed of L broth containing 100 μg/ml diamonopimelic acid, 50 μg/ml thymidine and 15 μg/ml tetracycline, and incubated at 37°C for two days.

(5-5) Four hundred and thirty two colonies appeared were divided into 18 groups, each containing 24 different bacterial clones, inoculated in 200 ml of L-broth containing 100 μg/ml of diaminopimelic acid, 50 μg/ml of thymidine and 10 μg/ml of tetracycline and cultured in shaking at 37°C for 5 to 7 hours. Then 200 ml of fresh L-broth containing chloramphenicol at a final concentration of 170 μg/ml was added to culture further for an overnight. Thus amplified plasmid DNA was purified according to a conventional mean. Clones possessing IL-2 cDNA were screened by a mRNA hybridization-translation assay (hereinafter "H-T" assay"). H-T assay here employed was carried out as follows:

Purified DNA (25 μg) was cleaved with restriction enzyme Hind III, treated with phenol three times, treated with phenol-chloroform and with chloroform, respectively, precipitated with ethanol, washed with

80% ethanol and dissolved in 40 µl of 80% formamide. The reaction mixture was heated for denaturation at 90°C for 5 min., then diluted to 1.3ml with 10 x SSC (1.5 M NaCl, 0.15 M sodium citrate). The DNA was thereafter fixed onto nitrocellulose filters, which filters were dried up at 80°C for 3 hrs and incubated for 18 hrs at 37°C in the solution containing 50% formamide, 20 mM Pepes of pH 6.5, 0.75 M NaCl, 5 mM EDTA, 0.2% SDB and 250 µg of poly (A) mRNA from induced J-111 cells to hybridize the DNA fixed on filters with IL-2 mRNA. Then the filters were washed at 65°C three times with solution consisting of 10mM Pipes of pH 6.5, 0.15 M NaCl, 1 mM Pipes, 10 mM NaCl solution and treated with 0.5 mM EDTA, 0.1% SDS solution at 95°C for 1 min to recover the hybridized mRNA from the filters. Thus extracted mRNA was purified on oligo dT-Cellulose column according to the conventional methods and injected into Xenopus oocytes to determine the IL-2 activity of translated proteins. One out of 18 groups, each consisting of 24 clones, gave positive 48 unit/ml IL-2 activity in $^3$H-TdR incorporation assay described previously, while others being clearly negative. Then 24 single colonies belonging to the positive group were inoculated in 200 ml of L-broth possessing the same composition described, cultured aerobically for 5 to 7 hrs. at 37°C and similarly chloramphenicol containing fresh L-broth was further added. After amplification of plasmid DNA by an overnight culture, plasmid DNA was similarly purified according to the standard procedures. After cleavage of about 5µg of each plasmid DNA with Hind III, each plasmid DNA was bound to nitrocellulose filters similarly. The filters were hybridized with I L-2 mRNA and hybridized mRNA was recovered to inject into Xenopus oocyte to determine the IL-2 activity of translated proteins.

As shown in Table 2, only plasmid DNA purified from a single colony, designated as p3-16, gave the

positive ILr2 activity. Therefore this clone was identified as the clone possessing ILr2 cDNA (E.coli x 1776/p3-16 AJ 11995 (FERM-BP-225)). Thus plasmid DNA, p3-16, was confirmed to share exactly the DNA (Il-2 gene) capable of forming the specific hybrid with IL-2 mRNA.

The CDNA insert of plasmid p3-16 showed characteristics to be cleaved by restriction enzyme XbaI at a single site and by BstNi at two sites, (at upstream and downstream of XbaI cleavage site). However the plasmid p3-16 contained a cDNA insert consisting of about 650 base pairs, which apparently corresponds to a part of IL-2 mRNA of 11 to 12S size. Therefore another cDNA library were prepared according to the procedure of Land et al. (Land et al., Nucleic Acids Res., vol 9 p2551, (1981)) using IL-2 mRNA as a template. Single stranded cDNA (1.6 µg) was synthesized by using 4µg of IL-2 mRNA elongated by dCMP residues, and ds-cDNA was synthesized by using oligo (dG) 12-18 as the primer for DNA polymerase I (Klenow fragment). The cDNA (0.6µg) longer than 680-base pair DNA size marker was obtained by a sucrose gradient cenrifugation and inserted into the PstI site of pBR322 by the standard G-C tailing method. After transformation of E. coli x 1776 by the recombinant DNA, approximately 2,000 colonies were screened by in situ hybridization method of Grunstein-Hogness with nick-translated p3-16 cDNA insert as the probe and the colony containing plasmid pIL 2-50A containing around 850 base pairs and the transformed clone (E. coli x 1776/pIL 2-50A, AJ 11996 (FERM-BP 226)) were identified. A restriction endonuclease cleavage maps of the cDNA insert of pIL 2-50A are shown in Fig. 1. To isolate a gene coding for IL-2 peptide from transformed E.coli x 1776 pIL 2-50A, plasmid DNA was digested with restriction enzyme PstI after isolation of DNA region from the cells according to the conventional means. Thus produced smaller fragment among generated two DNA fragments was DNA gene coding for IL-2 peptide. The complete nucleotide sequence of the PstI insert from pIL 2-50A was determined by the procedure of Maxam and Gilbert (Maxam, A.W. et al., Enzyme. 65, 499-560, 1980), and the whole structure is shown in Figure 2.

B, Preparation of gene coding for IL-2 polypeptide-(2)

A plasmid which should direct the synthesis of human IL-2 in E. coli cells was constructed as follows. A plasmid pTIL2-22 was constructed from pTrS-3 (Nishi T., Taniguchi T. et al., SEIKAGAKU 53, 967, (1981)), and pIL 2-50A containing the IL-Z cDNA by a series of modification procedures as illustrated in Fig. 4 (a). A plasmid pTrS-3 include insertion of the region of Trp promoter and Shine Dalgarno (hereinafter "SD") between EcoRi site and ClaI site of pBR322. The plasmid also contains an ATG initiation codon 13 bp downstream of the SD sequence as well as a single SphI site as illustrated in Figure 3. The vector is very efficient to produce the said protein when DNA sequence corresponding to the said protein is inserted in phase just downstream of the ATG codon, which is generated by SphI digestion and by subsequent treatment by T4 DNA polymerase of pTrS-3. Therefore the plasmid pTrS-3 (30 μg) was cleaved with a restriction enzyme SphI in a conventional manner and after successive treatment with phenol and chloroform, ethanol precipitates were recovered, then both ends were rendered flush by the treatment of T4 DNA polymerase. Then the DNA (21.4 μg) was recovered by similar successive phenol, chloroform treatment and ethanol precipitation. On the other side, 380 μg of pIL 2-50A containing an IL-2 cDNA was cleaved by PstI and the IL-2 cDNA insert was isolated by agarose gel electrophoresis. CDNA Insert (11 μg) was cleaved by HgiAI, treated by T4 DNA polymerase and 10 µg of the DNA of larger site was isolated by agarose gel electrophoresis. According to the procedures a cDNA (7.2 µg) coding for 132 amino acids was obtained and this DNA fragment had blunt ends (Fig. 4 (a)). Then the thus obtained cDNA fragment ligated to a pTrS-3 vector, previously digested by SphI and treated by T4 DNA polymerase just downstream of ATG sequence. Thus ligated plasmid was then used to transform into E. coli. HB 101 according to the conventional proceures. Ligation was carried out as follows. IL-2 cDNA (0.4 µg) larger fragment and 0.2 µg of pTrS-3 vector DNA were mixed with 0.8 unit of T4 DNA ligase in 66 mM Tris-HCl of pH 7.5 containing 6.6 mM $MgCl_2$, 1 mM ATP and 10 mM DTT, and the mixture was allowed to react at $4^O$C overnight. Among the transformants appeared on L broth agar plate containing ampicillin, colonies containing the IL-2 cDNA portion, which encodes 132 amino acids were selected by in situ colony hybridization assay. Thus selected colonies were cultured (10 ml) again to prepare plasmid DNA by lysozyme treatment and by freeze-thawing. The plasmid DNAs were cleaved with PstI and XbaI, and the resulting products were analysed by agarose gel electrophoresis in order to identify pTIL 2-22 in which the cDNA was linked to the ATG sequence of pTrS-3 in correct orientation. The E. coli. HB101 containing pTIL 2-22 was cultured under the conventional conditions known for the propagation of microorganisms. The cells were grown in 10 ml of x broth (2.5% Bactotrypton, 1.0% yeast extracts, 0.1% glucose, 20 mM $MgSO_4$, 50 mM Tris-HCl, pH 7.5) containing 25 µg/ml streptomycin and 25 µg of ampicillin at $37^O$C for an overnight. One ml of the culture suspension was inoculated into the same x broth (100 m2) and cultured at $37^O$C). When O.D. at 650 mµ arrived around 1.5-2.0 3-indole acrylic acid (IAA) was added. Three hours after the additon of inducer, the cells were collected, washed with 20 mM Tris-HCl (pH 7.5, 30mM NaCl) and resuspended into 8 ml of the same buffer. For the efficient functioning of Trp promoter inducers such as IAA was added at a final concentration of 50 µg/ml. Thus produced proteins in bacterial cells were extracted by sonication ($0^{\circ}$C 2 min.) or lysozyme (8 µg) digestion ($0^{\circ}$C, 20 min) followed with three successive freeze-thawing. According to this procedures IL-2 was usually extracted from organisms. The extracted IL-2 activity ranged from 10.000 to 120,000 units/ml.

E. coli HB101 containing pTIL 2-22 (AJ 12009) has been deposited in the accession number of FERM-BP 245.

C, Preparation of gene coding for IL-2 polypeptide-(3)

A plasmid which should direct the synthesis of human IL-2 in E. coli cells was constructed as follows. A plasmid pTIL2-21 was constructed from pTrS-3 (Nishi T., Taniguchi T: et al., SEIKAGAKU 53, 967, (1981)), and pIL 2-50A containing the IL-Z cDNA by a series of modification procedures as illustrated in Fig. 4(b). A plasmid pTrS-3 include insertion of the region of Trp promoter and Shine Dalgarno (hereinafter "SD") between EcoRI site and ClaI site of pBR322.

Plasmid pTrS-3 (10 µg) was at first cleaved with the restriction enzyme SalI and the SalI site was rendered flush by the treatment with DNA polymerase (Klenow fragment) or with T4 DNA polymerase. After cleavage with ClaI, a larger fragment, containing the trp promoter region, was isolated by agarose gel electrophoresis in a conventional manner to recover 3 µg of DNA.

On the other side, 11 µg of pIL 2-50A insert into PstI was cleaved with HgiAl, treated with T4 DNA polymerase and a larger fragment was isolated and purified by agarose gel electrophoresis. Thus cDNA fragment coding for 132 amino acids of IL-2 was obtained

**0142268**

in an amount of 7.2 µg. Then 0.45 µg of the fragment containing a trp promoter (described above) 0.5 µg of HgiAl-PstI fragment containing IL-2 cDNA and synthetic oligonucleotides (5') CGATAAGC TATGGCA (3'), and (3') TATTCGATACCGT (5') (each 20 pmole), both of which were phosphorylated at 5'-terminus, were ligated with 1.0 units of T4 DNA ligase in 66 mM Tris-HCl of pH 7.5 containing 6.6 mM MgCl$_2$, 1 mM ATP and 10 mM DTT, and the mixture was allowed to react at 4$^O$C overnight.

Thus ligated plasmid was then used to transform E. coli HB101. Among the transformants appeared on L broth agar plate containing ampicillin, the target transformants were selected as follows.

The candidate transformants able to hybridize with both of IL-2 cDNA and synthetic oligonucleotides were firstly selected by colony hybridization method, then the transformants possessing the insertion of DNA fragment initiating from CCT sequence at position III to 113 in Fig 2(a) (CCTACT -----) just downstream of ATG GCA sequence were selected by PstI,XbaI cleavage.

The E. coli HB 101 containing pTTL2-21a or pTTL2-21b was cultured under the conventional conditions known for the propagation of microorganisms. The cells were grown in 10 ml of x broth (2.5% Bactotrypton, 1.0% yeast extracts, 0.1% glucose, 20 mM MgSO$_4$, 50 mM Tris-HCl, pH 7.5) containing 25 µg/ml streptomycin and 25 µg of ampicillin at 37$^O$C for an overnight .One ml of the culture suspension was inoculated into the same x broth (100 ml) and cultured at 37$^O$C. When O.D. at 650 mµ arrived around 1.5-2.0 3-indole acrylic acid (IAA) was added. Three hours after the addition of inducer, the cells were collected, washed with 20 mM Tris-HCl (pH 7.5, 30 mM NaCl) and resuspended into 8 ml of the same buffer. For the efficient functioning of Trp promoter inducers such as IAA was added at a final concentration of

0142268

50 µg/m . Thus produced proteins in bacterial cells were extracted by sonication (0°C 2 min) or lyzozyme (8 µg) digestion (0°C, 20 min) followed with three successive freeze-thawing. According to this procedure IL-2 was usually extracted from organisms. The extracted IL-2 activity ranged from 10,000 to 120,000 units/ml.

Escherichia coli HB101 possessing pTIL2-21a (AJ 12013) and Escherichia coli HB101 possessing pTIL2-21b (AJ 12014) have been deposited in the assession numbers of FERM-BP 248 and FERM-BP 249 respectively.

D. Construction of yeast capable of producing IL-2

A yeast-E.coli shuttle vector pAM82 is carrying markers of arsl, 2µm ori and leu 2, and the promoter for the yeast of acid phosphatase (APase) gene. They also carry a 3.7 kb DNA segment of pBR322 which contains an ampicillin resistance marker (Ap$^\gamma$) and the origin of replication (Fig 5). The APase promoter is inducible by shifting phosphate concentration in culture medium from high into low.

Recombinant DNA pTIL2-21a(1µg) previously digested by BalI and XhoI linker (10 pmoles) previously phosphorylated at 5'-terminus were ligated overnight at 4°C with 1 unit of T$_4$ DNA ligase in 66mM Tris-HCl of pH 7.5 containing 6.6mM MgCl$_2$, 1mM ATP and 10mM DTT. Thus ligated DNA was the incorporated into E. coli HB101, and the transformant carrying plasmid (pTIL2-X16) which had XhoI site instead of Bal I site was selected. Next, 1µg of pTIL2-X16 previously digested by Hpa I, and XhoI linker (10 pmoles) were ligated with 1 unit of T$_4$ DNA ligase in the same manner as described above.

The ligated DNA was incorporated into E. coli and the transformant carrying plasmid (pTIL2-X2) which had XhoI site instead of HpaI site was selected

Plasmid pTIL2-X2 (30µg) was cleaved with restriction enzyme XhoI, and XhoI fragment (ca 1.6 Kb) containing IL-2 cDNA was isolated and purified by agarose gel electrophoresis to recover 5µg of DNA. Yeast-E. coli shuttle vector pAM82 (2µg) was cleaved similarly by XhoI, and the cleaved pAM82 and 2µg of the XhoI fragment were mixed in 66mM Tris-Hcl of pH 7.5 containing 1 unit of $T_4$ DNA ligase 6.6mM $MgCl_2$, 1mM ATP and 10mM DTT, and the mixture was held at $4^O$C overnight.

Thus ligated plasmid was then used for transformation into E. coli HB101. Among the transformants appeared on L broth agar plate containing ampicillin, colonies containing the IL-2 cDNA portion, which encodes 133 amino acids were selected by the colony hybridization assay mentioned before.

Each of the colonies slected was cultured to obtain cells and plasmid DNA in the cells were separated. The desired recombinant DNA should have about 7 kb XbaI fragment and about 2.5 kb XbaI fragment, and then the desired recombinant DNA was selected as that which formed about 7 kb XbaI fragment and about 2.5 Kb XbaI fragment by XbaI digestion, obtaining pYIL2-21a, pYIL2-21b, in which the cDNA was located at the position downstream of the acid phosphatase promoter in pAM82.

Next, the obtained plasmids pYIL2-21a and pYIL2-21b were introduced into Saccharomyces cerevisiae AH22 as follows. Cells of S. cerevisiae AH22 (a leu2, his4, can 1, $cir^+$) were aerobically cultured in 100ml of YPD medium (1% yeast extract, 2% polypepton, 2% glucose pH 5.3) placed in a 500 ml shaking flask at $30^O$C with shaking. At the middle of exponential growth phase (optical density at 610 nm was 0.8), the cells were harvested by centrifugation at 3,500 rpm for 5 min, washed once with TE buff (1mM Tris-HCl, pH 7.5, 0.1m MEDTA) and suspended in 5ml of the same buffer. A 0.5ml portion of this cell suspension was transferred to a test tube, an equal volume of 0.2M CH₃ COOLi or 1.0M Cscl was added to the suspension, and thereafter the suspension was held at 30° with shaking for one hour. The cells in suspension were harvested by centrifugation at 3,500 rpm for 5 min, washed once with 1 M sorbitol, and suspended again in 1ml of Zymolyase 5000 solution (1M sorbitol, 0.1M citrate buffer-10mM EDTA-0.4mg/ml zymolyase 5000). Protoplasts were prepared by shaking the suspension gently at 37°C for 1 hour. Then the protoplasts were washed three times with 1M sorbitol and resuspended in 1.0 ml of 1m sorbitol-10mM $CaCl_2$ - 10mM Tris-HCl, of pH 7.5. The plasmid DNA was added to the suspension of protoplasts to contain 20µg/ml and the mixture were incubated for 5 min. at a room temperature. Then, 5ml of 40% polyethylene glycol 4000-10mM $CaCl_2$ - 10mM Tris-HCl, of pH 7.5 was added to the mixture. After 10 min, the protoplasts were sedimented by centrifugation and resuspended in 5ml of the 1M sorbitol-10mM $CaCl_2$- 10mM Tris-HCl of pH 7.5. 0.2ml aliquots were added to 10ml of regeneration agar and poured on minimal agar plates consisting of 1M sorbitol, 0.67% Difco yeast nitrogen base, 2% glucose amino acid mixture solution containing each 5mg/dl of L-Glu, L-Asp, L-Ala, L-Pro, L-Thr, L-Ser. L-Trp, L-Ile, L-Val, L-Phe, L-Tyr, L-Met, gly, L-Gln, L-Asn, L-Lys, L-Cys, L-His and L-Arg and 2% Agar, pH 5.3 and Leu⁺ transformants were selected.

AH22 containing pYIL2-21a or pYIL2-21b was aerobically cultured at 30°C in 100ml of high-phosphate Burkholder minimal medium (containing 20.0g/l glucose, 2.0g/l asparagine, 1.5g/dl $KH_2PO_4$, 2.0g/l $(NH_4)_2SO_4$, 0.5g/l $MgSO_4 7H_2O$, 0.33 g/l $CaCl_2.2H_2O$, 0.1mg/l KI, 0.25 mg/l $FeSO_4$ $7H_2O$, 0.04 mg/l $MnSO_4 4H_2O$, 0.02mg/l $(NH_4)_6$ $MO_7O_{28} 4H_2O$, 0.50 mg/l $H_3BO_3$, 0.04 mg/l $CuSO_4.5H_2O$, 0.31 mg/l $ZnSO_4 7H_2O$, , 0.20 mg/l thiamine HCl, 10.0mg/l inositol, 0.2 mg/l Ca-pantothenate, 0.2 mg/l pyridoxine HCl, 0.05 mg/ml p-aminobenzoic acid, 9. 20 mg/l nicotinic acid and 2.0 $\gamma$/l biotin of pH 5.3) supplemented with histidine (20µg/ml). When the cell density reached to 0.4 in optical density at 610nm, a sample was taken out, centrifuged, and suspended for induction in 100 ml of low-phosphate minimal medium (containing 20.0g/l glucose, 2.0g/l asparagine, 1.5g/l KCl, 2.0g/l $(NH_4)_2SO_4$, 0.5g/l $MgSO_4.7H_2O$, 0.33g/l $CaCl_2 2H_2O$, 0.1 mg/l KI, 0.25mg/l $FeSO_4 7H_2O$, 0.04mg/l $MnSO_4.4H_2O$, 0.02mg/l$(NH_4)_6Mo_7O_{24}.4H_2O$, 0.6mg/l $H_3BO_3$, 0.04mg/l $CuSO_4.5H_2O$, 0.31mg/l $ZnSO_4.7H_2O$, 0.2 mg/l thiamine. HCl, 10.0mg/l inositol, 0.2 mg/l Ca-pantothenate, 0.2mg/l pyridoxine.HCl, 0.05 mg/l p-amino benzoic acid, 9.2 mg/l nicotinic acid and 2.0 $\gamma$/l biotin, of pH 5.3). Control samples were treated similarly except that high-phosphate minimal medium was used. When the density reached to 0.8 in optical density at 610nm, 100ml samples of the induced and noninduced cultures were centrifuged. The cells obtained were suspended in 5ml of a solution

containing Zymolyase 500 (100μg/ml), 1.2M sorbitol, 50mM phosphate (pH 7.2) and 14mM 2-mercaptoethanol, and the suspension were incubated for 30 min at 30 °C. The protoplasts were collected by centrifugation and lysed in 1 ml of 0.1% Triton x-100/50mM phosphate (pH 7.2) and 1mM phenylmethylsulphonyl-fluroide. The lysate was clarified by centrigugation at 11,000 rpm for 20 min. The IL-2 activity in the cleared lysate of low phosphate induced sample ranges from 1,000 to 10,000 units/ml. No IL-2 activity is found in high phosphate induced sample.

Saccharomyces cerevisiae AH22 possessing pYIL2-21a (AJ 14613) and Saccharomyces cerevisiae AH22 possessing pYIL2-21b (AJ 14614) were deposited at the Fermantation Research Institute, Japan, on the 18th October 1983, under the accession numbers FERM-P 7303 and FERM-P7304 respectively. Under the Budapest Treaty, these deposits were given the accession numbers FERM-BP 626 and FERM-BP 627, respectively, on the 2nd October 1984.

The characteristics of Saccharomyces cerevisiae AH22, which is a known strain, are disclosed in Proc. Natl. Acad. Sci. USA, 80, 1-5(1983) and in Proc. Natl. Acad. Sci. USA, 75, 1929-1933 (1978), and Saccharomyces cerevisiae FERM-7303 and Saccharomyces cerevisiae FERM-P 7304 have the same characteristics as Saccharomyces c.erevisiae AH22.

-36-   0142268

## Table 1

### (a)

| Sample | Dilution | Uptake of $^3$H-TdR (cpm) | Amount of IL-2* (unit/ml) |
|---|---|---|---|
| Control I | – | 553 | 0 |
| (Medium for assay) | | | |
| Control II | x 2 | 590 | 0 |
| (Supernatant of egg culture non-treated) | x 32 | 572 | |
| Translation product of fraction 13 | x 8 | 14,683 | 32 |
| | x 32 | 10,165 | |

### (b)

| | Dilution | Cell number of T-lymphocyte (No./well) | Amount of IL-2* (unit/ml) |
|---|---|---|---|
| Control I | x 2 | 0 | 0 |
| (Medium for assay) | x 16 | 0 | |
| Control II | x 2 | 0 | 0 |
| (Supernatant of egg culture non-treated) | x 16 | 0 | |
| Translation product of fraction 13 | x 2 | 115 | 40 |
| | x 16 | 55 | |

* The unit was calculated by comparing the amount of incorporated $^3$H-TdR with that of standard IL-2 (10 unit/ml) according to probit analysis.

Table 2

(a)

| Sample | Dilution | Uptake of $^3$H-TdR (cpm) | Amount of IL-2 (unit/ml) |
|---|---|---|---|
| Control I | − | 2,010 | 0 |
| (Medium for assay) | | | |
| Control II | x 2 | 2,120 | |
| (Supernatant of culture liquid of non-treated egg) | x 32 | 2,482 | 0 |
| Translation product of mRNA | x 2 | 20,453 | 58 |
| | x 32 | 20,961 | |

(b)

| Sample | Dilution | Cell number of T lymphocyte (cells/well) | Amount of IL-2 (unit/ml) |
|---|---|---|---|
| Control I | − | 0 | 0 |
| (Medium for assay) | | | |
| Control II | x 2 | 0 | 0 |
| (Supernatant of culture liquid of non-treated egg) | x 32 | | |
| Translation product of mRNA* | x 2 | 88 | 32 |
| | x 32 | 42 | |

* mRNA hybridized with cDNA from plasmid p3-16.

0142268

CLAIMS

1. A cell of Saccharomyces cerevisiae transformed with a recombinant DNA comprising a gene coding for a polypeptide possessing the activity of interleukin-2 and a vector DNA capable of propagating in said cell, the coding sequence of said gene being located at a position downstream of a promoter sequence.

2. The cell of claim 1, wherien said gene is prepared with a messenger RNA produced by an interleukin-2 producing mammalian cell.

3. The cell of claim 2, wherein said mammalian cell is a human T-lymphocyte, a transformed human T-lymphocyte or a T-cell hybridoma.

4. The cell of claim 2, wherein said messenger RNA is obtainable as a sediment of 11 to 12S of sucrose density gradient centrifugation.

5. The cell of claim 2, wherein said gene has sites cleaved with restriction endonuclease in the order of Bst NI, Xba I and Bst NI from 5'-terminus of the coding sequence.

6. The cell of claim 1, wherein said gene has sites cleaved with restriction endonuclease in the order of Dde I, Hinf I, Bst NI, Xba I, Bst NI and Sau 3A from 5'-terminus of the coding sequence.

7. The cell of claim 1, wherein said gene has the base sequence shown in Figure 2(a).

8. The cell of claim 4, wherein the base sequence of said gene corresponds to Amino Acid Sequence II in Figure 2 (b).

9. The cell of claim 4, wherein the base sequence of said gene corresponds to Amino Acid Sequence III in Figure 2 (b).

10. A method for producing interleukin-2 which comprises culturing Saccharomyces cerevisiae in culture medium, transformed with a recombination DNA to produce interleukin-2 and recovering the produced interleukin-2, said recombinant DNA comprising a gene coded for a polypeptide which possesses the activity of interleukin-2 and a vector DNA which is capable of replicating in said cell, and the coding sequence of said gene being located at a position downstream of a promoter sequence.

11. A method according to claim 11, wherein the Saccharomyces cerevisiae is that according to any of claims 1 to 9.

12. Saccharomyces cerevisiae FERM-P 7303.

13. Saccharomyces cerevisiae FERM-P 7304

## Fig. 1.

## Fig. 3.

## Fig. 2(a).

```
                                        1
                                        Met Tyr Arg Met GlN leu Leu Ser Cys Ile Ala
ATCACTCTCTTTAATCACTACTCACAGTAACCTCAACTCCTGCCACA ATG TAC AGG ATG CAA CTC CTG TCT TGC ATT GCA
                                            50

                         20
Leu Ser Leu Ala Leu Val Thr AsN Ser Ala Pro Thr Ser Ser Ser Thr Lys Lys Thr GlN Leu GlN Leu
CTA AGT CTT GCA CTT GTC ACA AAC AGT GCA CCT ACT TCA AGT TCT ACA AAG AAA ACA CAG CTA CAA CTG
                         100

            40
Glu His Leu Leu Leu Asp Leu GlN Met Ile Leu AsN Gly Ile AsN AsN Tyr Lys AsN Pro Lys Leu Thr
GAG CAT TTA CTG CTG GAT TTA CAG ATG ATT TTG AAT GGA ATT AAT AAT TAC AAG AAT CCC AAA CTC ACC
150                                                                  200

            60                                                                              80
Arg Met Leu Thr Phe Lys Phe Tyr Met Pro Lys Lys Ala Thr Glu Leu Lys His Leu GlN Cys Leu Glu
AGG ATG CTC ACA TTT AAG TTT TAC ATG CCC AAG AAG GCC ACA GAA CTG AAA CAT CTT CAG TGT CTA GAA
                                            250

                                                                            100
Glu Glu Leu Lys Pro Leu Glu Glu Val Leu AsN Leu Ala GlN Ser Lys AsN Phe His Leu Arg Pro Arg
GAA GAA CTC AAA CCT CTG GAG GAA GTG CTA AAT TTA GCT CAA AGC AAA AAC TTT CAC TTA AGA CCC AGG
            300                                                             350

                                                    120
Asp Leu Ile Ser AsN Ile AsN Val Ile Val Leu Glu Leu Lys Gly Ser Glu Thr Thr Phe Met Cys Glu
GAC TTA ATC AGC AAT ATC AAC GTA ATA GTT CTG GAA CTA AAG GGA TCT GAA ACA ACA TTC ATG TGT GAA
                                                    400

                                    140
Tyr Ala Asp Glu Thr Ala Thr Ile Val Glu Phe Leu AsN Arg Trp Ile Thr Phe Cys GlN Ser Ile Ile
TAT GCT GAT GAG ACA GCA ACC ATT GTA GAA TTT CTG AAC AGA TGG ATT ACC TTT TGT CAA AGC ATC ATC
                                    450

            153
Ser Thr Leu Thr
TCA ACA CTA ACT TGA TAATTAAGTGCTTCCCACTTAAAACATATCAGGCCTTCTATTTATTTAAATATTTAAATTTTATATTTATT
            500                                                 550

GTTGAATGTATGGTTTGCTACCTATTGTAACTATTATTCTTAATCTTAAAACTATAAATATGGATCTTTTATGATTCTTTTTGTAAGCCCT
                    600                                                     650

AGGGGCTCTAAAATGGTTTCACTTATTTATCCCAAAATATTTATTATTATGTTGAATGTTAAATATAGTATCTATGTAGATTGGTTAGTAA
                    700                                                     750

AACTATTT AATAAA TTTGATAAATATAAAAAAAAAAAAC - poly(A)
                        800
```

0142268

*Fig. 2(b).*

## Amino Acid Sequence I

Met Tyr Arg Met GlN Leu Leu Ser Cys Ile Ala Leu Ser Leu Ala Leu Val Thr AsN

Ser Ala Pro Thr Ser Ser Ser Thr Lys Lys Thr GlN Leu GlN Leu Glu His Leu Leu

Leu Asp Leu GlN Met Ile Leu AsN Gly Ile AsN AsN Tyr Lys AsN Pro Lys Leu Thr

Arg Met Leu Thr Phe Lys Phe Tyr Met Pro Lys Lys Ala Thr Glu Leu Lys His Leu

GlN Cys Leu Glu Glu Glu Leu Lys Pro Leu Glu Glu Val Leu AsN Leu Ala GlN Ser

Lys AsN Phe His Leu Arg Pro Arg Asp Leu Ile Ser AsN Ile AsN Val Ile Val Leu

Glu Leu Lys Gly Ser Glu Thr Thr Phe Met Cys Glu Tyr Ala Asp Glu Thr Ala Thr

Ile Val Glu Phe Leu AsN Arg Trp Ile Thr Phe Cys GlN Ser Ile Ile Ser Thr Leu

Thr

## Amino Acid Sequence II

Ala Pro Thr Ser Ser Ser Thr Lys Lys Thr GlN Leu GlN Leu Glu His Leu Leu Leu

Asp Leu GlN Met Ile Leu AsN Gly Ile AsN AsN Tyr Lys AsN Pro Lys Leu Thr Arg

Met Leu Thr Phe Lys Phe Tyr Met Pro Lys Lys Ala Thr Glu Leu Lys His Leu GlN

Cys Leu Glu Glu Glu Leu Lys Pro Leu Glu Glu Val Leu AsN Leu Ala GlN Ser Lys

AsN Phe His Leu Arg Pro Arg Asp Leu Ile Ser AsN Ile AsN Val Ile Val Leu Glu

Leu Lys Gly Ser Glu Thr Thr Phe Met Cys Glu Tyr Ala Asp Glu Thr Ile Val Glu

Phe Leu AsN Arg Trp Ile Thr Phe Cys GlN Ser Ile Ile Ser Thr Leu Thr

## Amino Acid Sequence III

Pro Thr Ser Ser Ser Thr Lys Lys Thr GlN Leu GlN Leu Glu His Leu Leu Leu Asp

Leu GlN Met Ile Leu AsN Gly Ile AsN AsN Tyr Lys AsN Pro Lys Leu Thr Arg Met

Leu Thr Phe Lys Phe Tyr Met Pro Lys Lys Ala Thr Glu Leu Lys His Leu GlN Cys

Leu Glu Glu Glu Leu Lys Pro Leu Glu Glu Val Leu AsN Leu Ala GlN Ser Lys AsN

Phe His Leu Arg Pro Arg Asp Leu Ile Ser AsN Ile AsN Val Ile Val Leu Glu Leu

Lys Gly Ser Glu Thr Thr Phe Met Cys Glu Tyr Ala Asp Glu Thr Ala Thr Ile Val

Glu Phe Leu AsN Arg Trp Ile Thr Phe Cys GlN Ser Ile Ile Ser Thr Leu Thr

# Fig. 4 A.

```
                                              20 21 22 23
                                              Ser Ala Pro Thr
pTrS-3   trp⇒ SD  ATGCATGC--          ---AGTGCACCTACT----
                  TACGTACG--          ---TCACGTGGATGA----   pIL2--50A

              │ SphI                         │ PstI, HgiAI
              ▼                              ▼

         trp⇒SD  ATGCATG               CCTACT----- CCCCCTGCA
                 TAC                   ACGTGGATGA----- GGGGG

              │ DNA pol. I Klenow           │ DNA pol. I Klenow
              │ or T₄ DNA pol.              │ or T₄ DNA pol.
              ▼                              ▼

         trp⇒ SD  ATG                  CCTACT----- CCCCC
                  TAC                  GGATGA------ GGGGG
```

T₄ DNA ligase

IL-2 cDNA

```
                    Met Pro Thr
                    ⌒⌒⌒ ⌒⌒⌒ ⌒⌒⌒
         trp⇒ SD   ATGCCTACT                    pTIL2-22
                   TACGGATGA


                                          Sal I ⟶

              Amp
```

# Fig. 4B.

pTrS—3
| SalI
| DNA pol. I Klenow
| ClaI

```
        trp ⇨ SD  ATI        TCGAC
                  TAGC        AGCTG
```

Amp

pIL2—50A
| PstI
| HgiAI
| DNA pol. I Klenow

ProThr
```
CCTACT-------CCCCC
GGATGA-------GGGGG
```

Synthetic oligomer
CGATAAGCTATGGCA

TATTCGATACCGT

T₄ DNA ligase

SD                          MetAlaProThr
```
trp ⇨ AAGGGTATCGATAAGCT ATGGCACCTACT-----
      TTCCCATAGCTATTCGA TACCGTGGATGA------
```
ClaI

pTIL2—21a

Amp                         PvuII

0142268

*Fig.5.*

EcoRI   HpaI

trp

IL-2

Ap'

pTIL2-21a

BalI

EcoRI

PvuII

BaLI

XhoI Linkers
T4 DNA Ligase

Hpai

XhoI Linkers
T4 DNA Ligase

EcoRI   XhoI

trp

IL-2

Ap'

pTIL2-X2

PvuII

XhoI

XhoI

EcoRI   ars 1

2μori

Ap'

pAM 82

leu 2

EcoRI

EcoRI

EcoRI

BamHI   Xho I

XhoI

T4 DNA Ligase

EcoRI

ars 1

2 ori

Ap'

leu 2

pYIL2-2l

IL-2

EcoRI   XhoI

BamHI

XhoI

XhoI        ATG                    XhoI
              IL-2

XhoI

BamHI(-700)                    -100

------------------------------TGAAACGTATATAAGCGCTGATGTTTTGCTAAGTCGAGGTTAGTAT

BstEII(-174)          Hogness box

-50

GGCTTCATCTCTCATGAGAATAAGAACAA-CCTCGAGGAACTAGTACGCAAGTTCACGTAAAAAGGG
Capping box                           Xho I

MetAlaProThr
TATCGATAAGCT ATGGCACCTACT------pBR322

## EUROPEAN SEARCH REPORT

European Patent
Office

| DOCUMENTS CONSIDERED TO BE RELEVANT | | | EP 84306934.5 |

| Category | Citation of document with indication, where appropriate, of relevant passages | Relevant to claim | CLASSIFICATION OF THE APPLICATION (Int. Cl 4) |
|---|---|---|---|
| X,P, D | EP - A1 - 0 091 539 (AJINOMOTO)<br><br>* Claims 1-7,18,22,24,25,27,29, 30 *<br><br>-- | 1-11 | C 12 N 15/00<br><br>C 12 P 21/02<br><br>//C 12 R 1:865<br><br>C 07 K 13/00 |
| A | EP - A2 - 0 077 571 (AJINOMOTO)<br><br>* Claims 1,2,5,7 *<br><br>---- | | |
| | | | **TECHNICAL FIELDS SEARCHED (Int. Cl.4)**<br><br>C 12 N<br><br>C 12 P<br><br>C 07 K |

The present search report has been drawn up for all claims

| Place of search | Date of completion of the search | Examiner |
|---|---|---|
| VIENNA | 22-01-1985 | FARNIOK |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

# DECLARATION PURSUANT TO RULE 28, PARAGRAPH 4, OF THE EUROPEAN PATENT CONVENTION

The applicant has informed the European Patent Office that, until the publication of the mention of the grant of the European patent or until the date on which the application has been refused or withdrawn or is deemed to be withdrawn, the availability of the micro-organism(s) identified below, referred to in paragraph 3 of Rule 28 of the European Patent Convention, shall be effected only by the issue of a sample to an expert.

## IDENTIFICATION OF THE MICRO-ORGANISMS

**Accession numbers of the deposits:**

FERM —BP  225
FERM —BP  226
FERM —BP  245
FERM —BP  248
FERM —BP  249
FERM —BP  626
FERM —BP  627